# EUROPEAN PATENT APPLICATION

(11) **EP 3 552 659 A1**
(43) Date of publication of application: **16.10.2019**
(21) Application number: 18166867.4
(22) Date of filing: 11.04.2018
(51) Int. Cl.: A61N 1/04, A61N 1/39

(54) **ELECTRODE PAD SET**

(71) Applicant: Koninklijke Philips N.V., 5656 AE Eindhoven (NL)
(72) Inventor: SECILMIS, Seckin Kemal, 5656 AE Eindhoven (NL); VERTATSCHITSCH, Dale, 5656 AE Eindhoven (NL); JONSEN, Eric, 5656 AE Eindhoven (NL)
(74) Representative: Steenbeek, Leonardus Johannes

(57) **Abstract**

An electrode pad set (12) comprises a plurality of pad electrodes (14), each electrode comprising an electrode gel, and lead wires (18) for electrically coupling a respective pad electrode of the plurality of pad electrodes to a base unit (15) configured to use the electrode pad set. A storage memory (17) stores data relating to a characteristic of the gel. The electrode pad set (12) further comprises a communication unit (16) configured to communicate the data to the base unit (15).

## Description

### FIELD OF THE INVENTION

The present embodiments relate generally to an electrode pad set, and a system comprising such an electrode pad set and a base unit, like a defibrillator, a TENS device, or a patient monitor.

### BACKGROUND OF THE INVENTION

US2004162586 discloses defibrillator electrodes with identification tags. A defibrillator uses electrical devices to query the tags and acquire electrode identification information. This document does not discuss gels.

Electrode sets or pads are used to measure biometric parameters or deliver electrical therapy to a patient (e.g., humans, animals, etc.). Electrodes of this type have a conductive layer that is applied to the skin. The conductive layer is typically coated with a gel-like material such as a hydro-gel which is designed to adhere to the skin and form an electrically conductive path between skin and conductive layer.

Conductive hydro-gels, however, have a finite shelf life because they slowly dry out or otherwise degrade over time. Once the gel loses its conductive properties, the electrode becomes unusable and the electrodes must be replaced.

Some defibrillators, such as the FRx automated external defibrillator (AED) manufactured by Philips, have replaceable electrodes pre-connected so that the AED can monitor the electrode condition. Such AEDs automatically and periodically self-test the electrode gel while the defibrillator is in a standby condition, i.e. prior to use. This is accomplished by measuring the complex impedance of the gel by passing an electrical current through the gel. If the measured impedance falls outside of an acceptable range, the gel fails the self-test and the defibrillator issues an alarm. The alarm prompts the user to replace the electrode. Typically, an electrode set may be replaced several times during the life of the AED, even if the electrode set is never deployed onto a patient.

Gel impedance varies greatly with temperature. The base unit must compensate for temperature during the self-test, generally by adjusting the acceptable impedance range for the gel as a function of temperature. One example of a test method is provided herein below.

| **Test Procedure:** |
|---|
| 1. Measure the magnitude of the complex impedance between the two back-to-back pads of the electrode pad set which are electrically connected together. Average this value over 10 samples. Call this value **PADS_Z**. |
| 2. If the test is run during the battery insertion test (BIT) only, then if PADS_**Z** is >5,000 Ohms or <240 Ohms, this test will produce a pads failure alert (WARN result). |
| 3. If the test is run during a daily (DPST), weekly (WPST), or monthly (MPST) periodic self-test only, then compare **PADS_Z** with the following table and produce a WARN result if **PADS_Z** exceeds the specified limit in the table or if **PADS_Z** < 240 Ohms. |

| **Temperature (degrees C)** | **PADS_Z Warn Limit** |
|---|---|
| <2 | 3420 |
| 2-4 | 3320 |
| 4-6 | 3226 |
| 6-8 | 3073 |
| 8-10 | 2929 |
| 10-12 | 2761 |
| 12-14 | 2572 |
| 14-16 | 2396 |
| 16-18 | 2216 |
| 18-20 | 2090 |
| 20-22 | 1969 |
| 22-24 | 1854 |
| 24-26 | 1745 |
| 26-28 | 1642 |
| 28-30 | 1544 |
| 30-32 | 1451 |
| 32-34 | 1364 |
| 34-36 | 1281 |
| 36-38 | 1202 |
| 38-40 | 1128 |
| 40-42 | 1059 |
| ≥42 | 992 |

The table of temperature versus electrode impedance limits of an electrode set in this prior art device is fixed in non-volatile memory, for example being hard-coded into non-volatile memory in the device operating software when the device is designed and manufactured. Thus it is very important that any electrode that could be attached to this prior art device has gel characteristics that match the self-testing parameters. Consequently, base units which use these electrode sets expressly rely upon acceptably low levels of gel variation within the population of all current and future electrode pad sets that could be connected to the device. Otherwise, a self-test alert may be issued when the gel is still usable, or (worse) a self-test alert may not issue when the gel is unusable.

Unfortunately, it is difficult to maintain tight control of manufacturing processes and material composition of the electrode sets throughout the typically multi-decade lifecycle of the underlying device. Gel manufacturers sometimes change or they go out of business. Gel manufacturers sometimes modify the gel manufacturing process. Gel ingredients may become unavailable or must be replaced with different performing materials. And gel manufacturing lots sometimes vary due to changing conditions at the manufacturing location. Any of these can change the gel characteristics in some way. Thus, if a future design change results in an electrode pad set with a different set of gel characteristics than that stored in the base unit, the base unit may sense the electrode pads sufficiency incorrectly.

### SUMMARY OF THE INVENTION

Accordingly, an improved electrode pad set, method and apparatus for overcoming the problems associated with gel characteristic variation in the art is desired and needed. The invention is defined by the independent claims. The dependent claims define advantageous embodiments. The inventors have discovered an improved technique to enable base units (e.g. patient monitors, defibrillators or TENS devices) to deal with electrodes that vary in gel characteristics. Instead of using a fixed table of characteristics for self-testing all electrodes to be connected to the base unit, the improved technique uses a table of characteristics that is custom to the attached electrode set and is disposed along with the electrode set. The base unit and electrode set are configured to communicate the custom table of characteristics into the base unit memory, such as non-volatile or commonly known rewritable memory, when the electrodes are connected to the base unit. The custom characteristic is then used for electrode self-testing. Each time the electrode set is replaced, a new custom table of characteristics may again be downloaded into the rewritable memory. Thus, the electrode self-testing limits are better matched to actual electrode gel characteristics. Additionally, the base unit is enabled to adjust itself to future changes in gel characteristics that may arise due to different electrode manufacturers.

In advantageous embodiments, each electrode carries with it, and conveys to the base unit, its relationship of the gel vs. temperature for self-diagnostic purposes. The base unit uses that custom relationship data to thereafter perform self-testing of the electrodes. When the electrode is replaced, a new custom relationship data set may be provided.

In accordance with one aspect of the present disclosure, an electrode pad set is disclosed which includes a capacity to communicate that electrode pad set's "critical to quality" characteristics. As used herein, the phrase "critical to quality" characteristics comprise at least an upper bound for at least one characteristic of the at least one pad electrode, and optionally a lower bound, wherein the upper and lower bounds are configured to ensure that a use of the at least one pad electrode stays within one or more predetermined specifications. The "critical to quality characteristics can further comprise one or more of an impedance vs. frequency table, impedance vs. temperature table, expiration date, serial number, batch code, date of manufacturing, and any operating specifications of the electrode pad set. In addition, devices which use the electrode pad set of the present disclosure are configured to be programmable in response to obtaining one or more critical to quality characteristics from an electrode set to thereby (i) advantageously accept the use of any electrode set with features according to the embodiments of the present disclosure and (ii) to no longer be bound by a specific manufacturer of an electrode pad set or product series, as will be better understood from the description presented herein.

In accordance with another aspect, a method of the present disclosure uses wireless communication, e.g., radio frequency identification (RFID) tags. In one embodiment, the electrode set is equipped with a passive (i.e., no energy required) wireless (RFID) tag programmed with critical to quality characteristics for at least one electrode of an electrode set. The critical to quality characteristics comprise the impedance vs. frequency characteristics, impedance vs. temperature characteristics, expiration date, serial number, batch code, date of manufacture, etc. According to one embodiment, a wireless (RFID) tag is disposed with at least one electrode of the electrode pad set, for example being adhered to an outer surface of the pad set. Devices which use the electrode pad sets such as defibrillators or patient monitors are configured to wirelessly exchange the critical to quality characteristics information of the electrode pad set. The device then uses the information to reprogram or adjust the corresponding device's signal analysis techniques to conduct self-testing of the electrode set, to reliably read biometric data such as an electrocardiogram (ECG) or properly deliver therapy from a defibrillation waveform.

In accordance with another aspect, a base unit which is connected to the electrode pad set can also be configured to warn users if any of the critical to quality characteristics deviate from specifications. In addition to the characteristics already mentioned herein above, the "critical to quality" characteristics also advantageously include upper and optionally lower bounds to ensure the electrode pad sets stay within specifications.

In another aspect, a method of communicating operating characteristics data of an electrode pad set includes providing disposable electrodes with a communicating unit (e.g. RFID tag or other suitable communication unit as discussed further herein) to pass electro-gel characteristics to a base unit, e.g. an automatic external defibrillator (AED) when connected. In response, the AED adjusts its parameters for self-testing and operating protocols based on the electro-gel characteristics. In one embodiment, the AED sends results of periodic gel testing (e.g., obtained during self-tests) back to a central service center (i.e., a remote service provider) for compiling lot data of the particular electrodes. As mentioned above, the electro-gel characteristics comprise critical to quality characteristics that include at least upper and optionally lower bounds to ensure the electrode pad sets stay within specifications.

According to one embodiment, an electrode pad set comprises a plurality of pad electrodes, each pad electrode including an electrode gel and lead wires having first and second ends. Each of the lead wires is electrically coupled between (i) a respective pad electrode of the plurality of pad electrodes at the first end and (ii) at least one electrical connector at the second end. The at least one electrical connector is configured to electrically couple the lead wires to a mating electrical connector of a medical device, for example, a defibrillator configured to use the electrode pad set.

The electrode pad set further includes a communication unit disposed adjacent the plurality of pad electrodes. More than one communication unit could be provided, e.g., one for each electrode pad. Still further, the electrode pad set further includes a data storage memory. The data storage memory is configured to store data relating to a relationship between a characteristic of the electrode gel and an environmental variable, as is discussed further in this disclosure. In operation, the communication unit is configured to communicate the data relating to a relationship between a characteristic of the electrode gel and an environmental variable stored in the data storage memory of the electrode pad set to device which is configured to use the electrode pad set.

In one embodiment, the data relating to a relationship comprises a table of values of the characteristic of the electrode gel as relating to the environmental variable. In another embodiment, the characteristic of the electrode gel is electrical impedance and the environmental variable is one selected from temperature, a test signal frequency, and time. In addition, the data relating to the relationship further comprises a threshold criterion for an acceptable operating specification of the electrode gel. In a further embodiment, the communication unit comprises one or more of a passive RFID tag, SPI, Dual SPI, Quad SPI, UART, I2C, Single Wire/1-wire, HSL, Parallel Flash, USB, NFC, RFID, Bluetooth, Fiber optic, Zigbee/ZWAVE, IRDA, and Wi-Fi. The communications unit may alternatively comprise providing the data to the device in a wired fashion via the connected electrode leads, using perhaps one of the technologies recited above. The listed communication techniques are well known in the art and not further discussed herein.

According to another embodiment, a system comprises an electrode pad set and a base unit. The base unit comprises a controller, a self-testing circuit under operational control of the controller and disposed to automatically and periodically test a readiness of the electrode pad set. The base unit further comprises a volatile data storage memory in communication with the self-testing circuit and configured to store data pertaining to a relationship between a characteristic of an electrode gel and an environmental variable. Preferably, the base unit volatile memory is relatively persistent and low-power, such as Flash memory, to which data may be transferred relatively easily.

In addition, the base unit includes a communications module corresponding to the electrode pad set communication unit. The communication module is further in communication with the volatile data storage memory, e.g., via a signal bus. The base unit still further includes a socket configured to receive a mating electrical connector so as to provide electrical connection between the electrode pad set and the self-testing circuit. In a further embodiment, the communication module comprises a communication device configured to communicate via one or more of a passive RFID tag, SPI, Dual SPI, Quad SPI, UART, I2C, Single Wire/1-wire, HSL, Parallel Flash, USB, NFC, RFID, Bluetooth, Fiber optic, Zigbee/ZWAVE, IRDA, and Wi-Fi. According to another embodiment, the defibrillator system further comprises a connection sensor configured to provide an electrode connection signal to the controller responsive to a sensed connection between the socket and the mating electrical connector. In operation, the controller is configured to initiate a data transfer from the electrode pad set data storage memory to the defibrillator base unit data storage memory, via the communication module, responsive to the electrode connection signal. For example, the electrode connection signal could be issued upon an initial coupling of the mating electrical connector with the socket. In other embodiments, the electrode connection signal could be issued intermittently to indicate a continued connection of the mating electrical connector with the socket, according to the requirements of a given defibrillator system or medical device.

As noted herein above, in operation, the controller initiates a data transfer from the electrode pad set data storage memory to the base unit data storage memory, via the communication module, responsive to the electrode connection signal. In one embodiment, subsequent to the data transfer, the self-testing circuit automatically and periodically tests the readiness of the electrode pad set using the transferred data.

In yet another embodiment, the base unit further comprises a battery connector sensor in communication with the controller. The battery connector sensor is configured to provide a battery connection signal responsive to a sensed installation of a battery into the system. According to one embodiment, in operation, the controller initiates a data transfer from the electrode pad set data storage memory to the defibrillator base unit data storage memory, via the communication module, responsive to the battery connection signal.

According to a still further embodiment, the system further comprises a visual indicator and an audio output, e.g., via a user interface and an audio output, in communication with the controller. In one embodiment, in operation, at least one of the visual indicator and the audio output provide an alarm responsive to a sensed failed electrode pad set readiness test via the self-testing circuit. In another embodiment, the transferred data relating to the relationship comprises a functional relationship to or a table of values of the characteristic of the electrode gel as relating to the environmental variable. Still further, subsequent to the data transfer, the data in the base unit volatile data storage memory is the same as the data in the electrode pad set data storage memory.

In another embodiment, the characteristic of the electrode gel is electrical impedance and the environmental variable is one selected from temperature, a test signal frequency, and time. The data relating to the relationship can further comprise a threshold criterion for an acceptable operating specification of the electrode gel. In a further embodiment, the base unit volatile data storage memory is further configured to store at least one of electrode pad set test failure threshold criteria and upper bound, and optionally a lower bound for an electrode gel critical to quality characteristic.

According to yet another embodiment, a method for self-testing an electrode pad set that is electrically connected to a base unit comprises the steps of: providing a system as discussed herein; sensing, via connection sensor, a connection of the mating electrical connector of the pad electrode set to the base unit; initiating communications, via the base unit communication module, between the base unit volatile data storage memory and the data storage memory of the electrode pad set responsive to the connection sensing step; writing the data from the data storage memory of the electrode pad set to the base unit volatile data storage memory; and subsequent to the writing step, performing an automatic electrode pad set self-test, via the self-testing circuit and the lead wires, using the data. In a next step, a query is performed as to whether the readiness of the pad electrodes is within limits per the data. If the readiness of the pad electrodes is within limits per the data, the method proceeds with devices operations, as appropriate, i.e., with knowledge that the pad electrodes are ready for use. On the other hand, if the query step resulted in a determination that the electrode pads fail the readiness self-test, i.e., the readiness of the pad electrodes is outside the limits per the data, then the method proceeds to produce a WARN result. Subsequent to the WARN result, the method continues, i.e., with knowledge that the pad electrodes are not ready for use and/or are defective and in need of replacement.

In another embodiment, the system further comprises a battery connector sensor in communication with the controller, the method further comprising the steps of: sensing, via the battery connector sensor, an installation of a battery into the system by the battery connector sensor; initiating communications responsive to the sensing step, via the base unit communication module, between the base unit volatile data storage memory and the electrode pad set data storage memory responsive to the battery installation sensing step; writing the data from the data storage memory of the electrode pad set to the base unit volatile data storage memory; and subsequent to the writing step, performing an automatic electrode pad set self-test, via the self-testing circuit and the lead wires, using the data. The method thereafter may continue to perform self-testing using the data at pre-determined intervals until the electrode pad is replaced.

In yet another embodiment, the system further comprises a display (e.g., user interface) in communication with the controller, the method further comprising the step of: providing an indication, via the display, that the writing step has occurred. According to one embodiment, the method further comprising the step of: transmitting a notification to a remote service provider that the writing step has occurred.

According to a still further embodiment, a base unit comprises: a controller; a self-testing circuit under operational control of the controller and disposed to automatically and periodically test a readiness of an electrode pad set; a volatile data storage memory in communication with the self-testing circuit and configured to store data pertaining to a relationship between a characteristic of an electrode gel and an environmental variable; and a communication unit configured to receive the data pertaining to a relationship between a characteristic of the electrode gel and an environmental variable from a connected electrode pad set and to store the data into the volatile data storage memory.

Still further advantages and benefits will become apparent to those of ordinary skill in the art upon reading and understanding the following detailed description.

### BRIEF DESCRIPTION OF THE DRAWINGS

The embodiments of the present disclosure may take form in various components and arrangements of components, and in various steps and arrangements of steps. Accordingly, the drawings are for purposes of illustrating the various embodiments and are not to be construed as limiting the embodiments. In the drawing figures, like reference numerals refer to like elements. In addition, it is to be noted that the figures may not be drawn to scale.
Fig. 1 is a functional block diagram view of a base unit and an electrode pad set for communicating operating characteristics data of the electrode pad set according to an embodiment of the present disclosure;
Fig. 2 is a functional block diagram view of a base unit and an electrode pad set for communicating operating characteristics data of the electrode pad set according to another embodiment of the present disclosure; and
Fig. 3 is a flow chart of a method for automatically self-testing a pre-connected electrode pad set with a defibrillator, using self-testing criteria data that has previously been communicated from the electrode pad set to the base unit.

### DESCRIPTION OF EMBOSIMENTS

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the embodiments of the present disclosure, which is defined solely by the appended claims and applicable law.

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein can be used in the practice or testing of the embodiments.

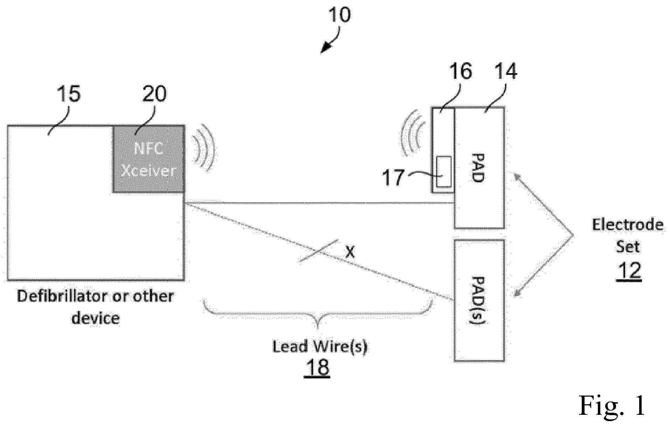

With reference now to Fig. 1, a functional block diagram view is shown of a system 10 comprising a base unit 15 and an electrode pad set 12 for communicating operating characteristics data of the electrode pad set according to an embodiment of the present disclosure. Base unit 15 can comprise, for example, a defibrillator, patient monitor, or other device as will be understood from the disclosure herein. The electrode pad set 12 comprises a plurality of electrode pads 14. At least one communication unit 16 (e.g., a Near Field Communications (NFC) tag) is coupled to, or integrated within, at least one electrode pad of the electrode pad set (or plurality of pad electrodes). In one embodiment, the NFC tag 16 may comprise a Radio Frequency Identification (RFID) tag, and more particularly, a passive RFID tag.

The electrode pad set 12 is provided with a corresponding set of lead wires 18, with at least one lead wire per electrode pad. The lead wires 18 each have first and second ends. Each of the lead wires is electrically coupled between (i) a respective pad electrode 14 of the plurality of pad electrodes 12 at the first end and (ii) at least one electrical connector (not shown) at the second end. In addition, the at least one electrical connector (not shown) at the second end is configured to electrically couple the lead wires 18 to a mating electrical connector (not shown) of a device 10 configured to use the electrode pad set. The base unit 15 also includes a communication module 20 (e.g., a Near Field Communications (NFC) transceiver) for wirelessly communicating and exchanging information with the communication unit 16 attached to, or integrated with, the at least one electrode pad 14.

In one embodiment, the at least one communicating unit 16 includes a data storage 17 that has stored therein at least one set of critical to quality characteristics of the at least one pad electrode 14 of the plurality of pad electrodes 12. The set of critical to quality characteristics can include upper and/or lower bounds for at least one of the critical to quality characteristics. The upper and lower bounds are selected to ensure that a use of the at least one pad electrode 14 performs within one or more predetermined specifications. In other words, the upper and lower bounds for at least one of the critical to quality characteristics comprise upper and lower bounds for at least one operating specification of the at least one pad electrode. In another embodiment, the set of critical to quality characteristics, for the at least one pad electrode of the plurality of pad electrodes, further includes one or more of an impedance vs. frequency table, and an impedance vs. temperature table. The characteristics may also comprise other quality-assurance-related data such as an expiration date, a serial number, a batch code, a date of manufacturing, and at least one operating specification of the at least one electrode.

As discussed herein, the at least one pad electrode 14 of the plurality of pad electrodes 12 comprises a gel layer having adhesive and electrically conductive characteristics. The adhesive and electrically conductive characteristics of the gel material may vary within the population of suitable pad electrodes due to material composition, manufacturing source, and because of different manufacturing processes. The electrically conductive characteristics include, for example, impedance vs. frequency and impedance vs. temperature characteristics of the at least one electrode pad. Preferably, the electrodes 12 are configured to be stored with the respective gel layers in electrical contact with each other and with the defibrillator. Thus a circuit may be formed to enable the defibrillator to automatically inject a test current and measure impedance of the current passing through the gel.

With reference still to Fig. 1, base unit 15 is configured for performing at least one of (i) obtaining diagnostic data (e.g., biometric data) from a patient (not shown) and (ii) delivering therapy to the patient (not shown), via the electrode pad set 12 according to the various embodiments of the present disclosure. In operation, responsive to the at least one electrical connector (not shown) of the electrode pad set 12 being electrically coupled to the at least one mating connector (not shown) of the base unit 15, the base unit 15 is operable to (i) obtain, via the wireless communications module 20 and the at least one communication unit 16, the critical to quality characteristics of the at least one pad electrode of the plurality of electrode pads, (ii) reprogram or adjust at least one signal analysis technique, self-testing, or operating protocol of a diagnostic unit (not shown) or therapy unit (not shown) of the base unit 15, in response to the obtained critical to quality characteristics of the at least one pad electrode 14 of the plurality of electrode pads 12, and (iii) perform at least one of (a) automatically self-test the electrode pads for sufficiency, (b) read biometric data, via the diagnostic unit, or (c) deliver therapy from a defibrillation waveform, via the therapy unit, based on the reprogrammed or adjusted at least one signal analysis technique, self-testing, or operating protocol.

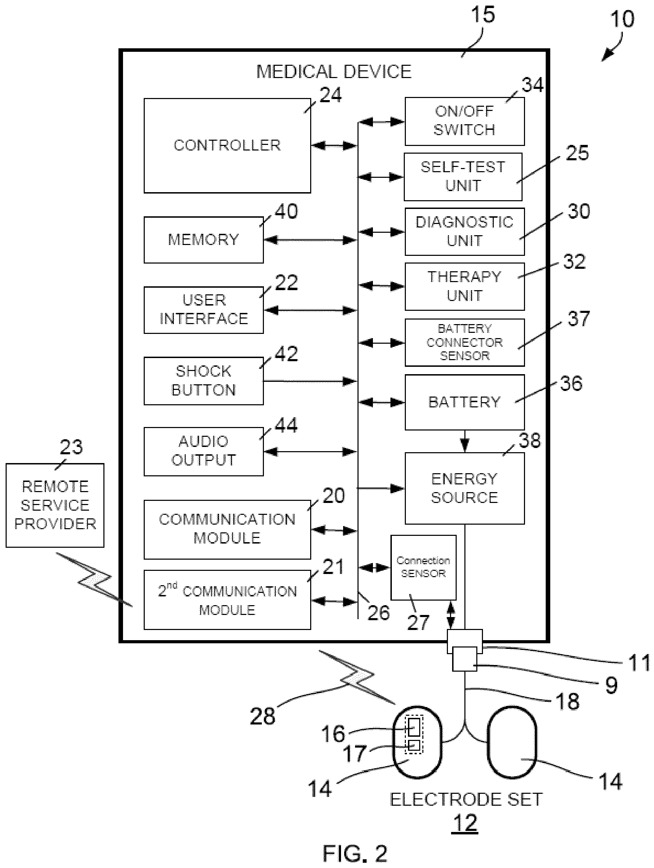

Turning now to Fig. 2, there is shown a functional block diagram view of a system 10 comprising a base unit 15 and an electrode pad set 12 for communicating operating characteristics data of the electrode pad set according to another embodiment of the present disclosure. The system 10 can comprise a portable medical device, for example, for use by a first responder to carry out at least one action at a rescue scene in connection with a subject needing emergency or other treatment according to an embodiment of the present disclosure. In one embodiment, the portable medical device 10 includes at least a user interface 22, a communications module 20, and a controller 24. The user interface 22 comprises any suitable user interface operatively coupled to at least the controller 24, via signal lines 26, for use in connection with one of a diagnostic or therapy during an emergency situation, as discussed herein. For example, user interface 22 can comprise at least one selected from the group consisting of an input/output device, a tactile output device, a touch screen, an optical display, a microphone, a keypad, a keyboard, a pointing device, an image capture device, a video camera, an audio output device, and any combination thereof, determined as appropriate according to the requirements of a given portable medical device implementation and/or application.

The communications module 20 is configured for wirelessly communicating, bi-directionally, with at least the communication unit 16 located within a given immediate proximity of the portable medical device 10. The communications module 20 is further for receiving, via the at least one communication unit 16 of the at least one pad electrode 14 of the plurality of pad electrodes 12, the critical to quality characteristics of the at least one pad electrode of the plurality of electrode pads. Communications module 20 is preferably a low-power short-range transceiver or wireless or wired connection, which communication is established only when the electrode is disposed in close proximity to the defibrillator.

Communication between the communication module 20 of the portable medical device 10 and the at least one communication unit 16 of the at least one pad electrode 14 of the plurality of pad electrodes 12 is indicated by reference numeral 28. In other words, communication between the various devices and components as discussed herein is preferably accomplished using suitable near-field communication techniques known in the art, and thus are not discussed further herein. The controller 24 operatively couples to the user interface 22 and the communication module 20 via suitable signal lines, indicated via reference numeral 26. Controller 24 is configured for operating, in response to the at least one electrical connector 9 of the electrode pad set 12 being electrically coupled to the at least one mating connector 11 of base unit 15, to (i) obtain, via the wireless communications module 20 and the at least one communication unit 16, the critical to quality characteristics of the at least one pad electrode 14 of the plurality of electrode pads 12, (ii) reprogram or adjust at least one signal analysis technique, self-testing, or operating protocol of a diagnostic unit 30 or a therapy unit 32, in response to the obtained critical to quality characteristics of the at least one pad electrode of the plurality of electrode pads, and (iii) perform at least one of (a) automatically self-test the electrode pads for sufficiency, (b) read biometric data, via the diagnostic unit 30, or (c) deliver therapy from a defibrillation waveform, via the therapy unit 32, based on the reprogrammed or adjusted at least one signal analysis technique, self-testing, or operating protocol.

In one embodiment, controller 24 comprises one or more of a microprocessor, microcontroller, field programmable gate array (FPGA), integrated circuit, discrete analog or digital circuit components, hardware, software, firmware, or any combination thereof, for performing various functions as discussed herein, further according to the requirements of a given portable medical device implementation and/or application. Controller 24 can further comprise one or more of the various modules discussed herein.

With reference still to Fig. 2, the base unit 15 of portable medical apparatus 10 can further comprise one or more of an ON/OFF switch 34, the diagnostic unit or module 30, the therapy unit or module 32, a battery 36, an energy source 38, memory 40, shock button 42 (e.g., for activating the administration of a shock via AED pad electrodes), and audio output or module 44. Each of the one or more of the ON/OFF switch 34, diagnostic unit or module 30, therapy unit or module 32, battery 36, energy source 38, memory 40, shock button 42, and audio output module 44 is operatively coupled to at least the controller 24, e.g., via signal lines 26.

The ON/OFF switch 34 comprises any suitable switch for powering the portable medical apparatus 10 between ON and OFF. The diagnostic unit or module 30 comprises any suitable computer program module for rendering a diagnostic for a given implementation and/or portable medical device application. The therapy unit or module 32 comprises any suitable computer program module for rendering a therapy for a given implementation and/or portable medical device application. It is understood that the described modules may be computer program modules which are rendered in a non-transitory computer-readable medium.

In one embodiment, battery 36 can comprise any suitable power source or power supply for a given portable medical device implementation and/or application. In addition, energy source 38 can comprise any suitable power source or power supply for a given portable medical device implementation and/or application. For example, for a portable medical device comprising an AED device, the energy source 38 can comprise high voltage capacitor suitable for storing energy effective in defibrillating shocks, where the capacitor is charged by battery 36 through a charging circuit (not shown). Furthermore, memory 40 can comprise any suitable memory device, operatively coupled to at least the controller 24, for at least storing information thereto, and further for at least subsequently retrieving the information there from. Memory 40 is preferably a somewhat persistent and very low-power volatile memory, such as flash memory, to which the aforementioned data table can be automatically written, stored, and subsequently retrieved for use in an electrode self-test.

The portable medical apparatus 10 is further operable for use with a pair of AED pad electrodes 14 operatively coupled to energy source 38, for administration of an electrical shock during use of the portable medical device 10 as an AED device. At least one of the electrode pads 14 of the set or plurality of electrode pads 12 is operatively coupled via suitable communication link 28 (e.g., a near field communication (NFC), Radio Frequency Identification (RFID), or other suitable short-range communication link) with communication module 20 of the portable medical apparatus 10.

Referring again to Figs. 1 and 2, according to one embodiment, an electrode pad set 12 comprises a plurality of pad electrodes 14, each pad electrode including an electrode gel and lead wires 18 having first and second ends. Each of the lead wires 18 is electrically coupled between (i) a respective pad electrode of the plurality of pad electrodes 14 at the first end and (ii) at least one electrical connector 9 at the second end. The at least one electrical connector 9 is configured to electrically couple the lead wires 18 to a mating electrical connector 11 of a medical device 10, for example, a defibrillator configured to use the electrode pad set 12. The electrode pad set 12 further includes a communication unit 16 disposed adjacent the plurality of pad electrodes 14. In the illustration of Figure 2, only one communication unit 16 is shown; however, more than one communication unit could be provided, e.g., one for each electrode pad. Still further, the electrode pad set 12 further includes a data storage memory 17. The data storage memory 17 is configured to store data relating to a relationship between a characteristic of the electrode gel and an environmental variable, as is discussed further in this disclosure. In operation, the communication unit 16 is configured to communicate the data relating to a relationship between a characteristic of the electrode gel and an environmental variable stored in the data storage memory 17 of the electrode pad set 12 to base unit 15 which is configured to use the electrode pad set, as discussed further herein.

In one embodiment, the data relating to a relationship comprises a table of values of the characteristic of the electrode gel as relating to the environmental variable. In another embodiment, the characteristic of the electrode gel is electrical impedance and the environmental variable is one selected from temperature, a test signal frequency, and time. In addition, the data relating to the relationship further comprises a threshold criterion for an acceptable operating specification of the electrode gel. In a further embodiment, the communication unit 16 comprises one or more of a passive RFID tag, SPI, Dual SPI, Quad SPI, UART, I2C, Single Wire/1-wire, HSL, Parallel Flash, USB, NFC, RFID, Bluetooth, Fiber optic, Zigbee/ZWAVE, IRDA, and Wi-Fi. The listed communication techniques are well known in the art and not further discussed herein. Communication unit 16 may alternatively comprise transmitting data from the memory 17 to the device 10 via the electrode leads when the electrode is connected.

With reference still to Figs. 1 and 2, according to another embodiment, a defibrillator system 10 for delivering electro-therapy to a patient, comprises an electrode pad set 12 as discussed herein above and a defibrillator base unit 15. The defibrillator base unit 15 comprises a controller 24, a self-testing circuit 25 under operational control of the controller and disposed to automatically and periodically test a readiness of the electrode pad set. The base unit 15 further comprises a volatile data storage memory 40 in communication with the self-testing circuit 25 and configured to store data pertaining to a relationship between a characteristic of an electrode gel and an environmental variable. In addition, the base unit 15 includes a communication module 20 corresponding to the electrode pad set communication unit 16. The communication module 20 is further in communication with the volatile data storage memory 40, e.g., via signal bus 26. The base unit 15 still further includes a socket 11 configured to receive a mating electrical connector 9 so as to provide electrical connection between the electrode pad set 12 and the self-testing circuit 25. In a further embodiment, the communication module 20 comprises a communication device configured to communicate via one or more of a passive RFID tag, SPI, Dual SPI, Quad SPI, UART, I2C, Single Wire/1-wire,HSL, Parallel Flash, USB, NFC, RFID, Bluetooth, Fiber optic, Zigbee/ZWAVE, IRDA, and Wi-Fi.

According to another embodiment, the defibrillator system 10 further comprises a connection sensor 27 configured to provide an electrode connection signal to the controller 24 responsive to a sensed connection between the socket 11 and the mating electrical connector 9. In operation, the controller 24 is configured to automatically initiate a data transfer from the electrode pad set 12 data storage memory 17 to the defibrillator base unit 15 data storage memory 40, via the communication module 20, responsive to the electrode connection signal. For example, the electrode connection signal could be issued upon an initial sensed coupling of the mating electrical connector 9 with the socket 11. In other embodiments, the electrode connection signal could be issued periodically to indicate a continued connection of the mating electrical connector 9 with the socket 11, according to the requirements of a given defibrillator system or medical device.

As noted herein above, in operation, the controller 24 initiates a data transfer from the electrode pad set 12 data storage memory 17 to the defibrillator base unit 15 data storage memory 40, via the communication module 20, responsive to the electrode connection signal. In one embodiment, subsequent to the data transfer, the self-testing circuit 25 automatically and periodically tests the readiness of the electrode pad set 12 using the transferred data.

The inventors have recognized that an electrode set is sometimes connected to the defibrillator before the battery is installed, and thus the connection would not be detected. So it may be desirable to obtain the electrode pad data whenever a battery is sensed as attached as well. Therefore, in yet another embodiment, the defibrillator system 10 and defibrillator base unit 15 further comprises a battery connector sensor 37 in communication with the controller 24. The battery connector sensor 37 is configured to provide a battery connection signal responsive to a sensed installation of a battery 36 into the defibrillator system 10. According to one embodiment, in operation, the controller 24 initiates a data transfer from the electrode pad set 12 data storage memory 17 to the defibrillator base unit 15 data storage memory 40, via the communication module 20, responsive to the battery connection signal.

According to a still further embodiment, the defibrillator system 10 further comprises a visual indicator and an audio output, e.g., via user interface 22 and audio output 44, in communication with the controller 24. In one embodiment, in operation, at least one of the visual indicator and the audio output provide an alarm responsive to a sensed failed electrode pad set readiness test via the self-testing circuit 25.

Defibrillator system 10 may also be disposed with a second wireless communication module 21 that is disposed to notify a remote service provider 23 of activity related to the invention, such as that the data writing step has occurred, or that an electrode self-test has failed. In addition, a transmission from remote service provider 23 to defibrillator system 10 using the second wireless communications module 21 may be used to update the gel relationship table in a redundant path to the communications link 28. Second communications module 21 may be a wired communication via an internet, Ethernet, or telephonic network, or may be a wireless connection using a cellular telephonic connection with optionally one or more of wireless protocols as exemplified for communications link 28.

In another embodiment, the transferred data relating to the relationship comprises a table of values of the characteristic of the electrode gel as relating to the environmental variable. Still further, the defibrillator system 10 further includes wherein, subsequent to the data transfer, the data in the defibrillator base unit 15 volatile data storage memory 40 is the same as data in the electrode pad set 12 data storage memory 17.

In another embodiment, the characteristic of the electrode gel is electrical impedance and the environmental variable is one selected from temperature, a test signal frequency, and time. The data relating to the relationship can further comprise a threshold criterion for an acceptable operating specification of the electrode gel. In a further embodiment, the defibrillator volatile data storage memory 40 is further configured to store at least one of electrode pad set 12 test failure threshold criteria and upper and lower bounds for an electrode gel critical to quality characteristic.

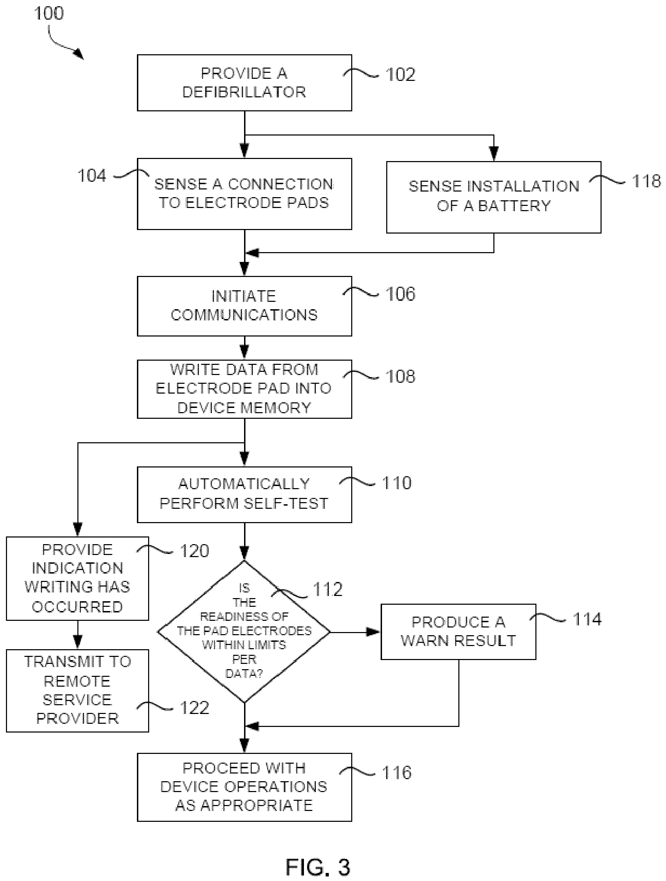

With reference now to Fig. 3, according to yet another embodiment, a method 100 for self-testing an electrode pad set that is electrically connected to a base unit 15 comprises the steps of: providing (Step 102) a system 10 as discussed herein; sensing (Step 104), via connection sensor 27, a connection of the mating electrical connector 9 of the pad electrode set 12 to the defibrillator base unit socket 11; initiating communications (Step 106), via the base unit communication module 20, between the base unit 15 volatile data storage memory 40 and the data storage memory 17 of the electrode pad set 12 responsive to the connection sensing step; and writing the data (Step 108) from the data storage memory 17 of the electrode pad set 12 to the base unit 15 volatile data storage memory 40. In practice, this portion of the method 100 is effective to automatically replace data pertaining to a particular electrode gel characteristic every time a new electrode is attached to the base unit 15.

Subsequent to the writing step, the method continues with the step of performing (Step 110) an automatic electrode pad set self-test, via the self-testing circuit 25 and the lead wires 18, using the data. In a next step, a query (Step 112) is performed as to whether the readiness of the pad electrodes is within limits per the data. If the readiness of the pad electrodes is within limits per the data, the method proceeds with devices operations, as appropriate (Step 116), i.e., with knowledge that the pad electrodes are ready for use. On the other hand, if the query step (Step 112) resulted in a determination that the electrode pads fail the readiness self-test, i.e., the readiness of the pad electrodes is outside the limits per the data, then the method proceeds to produce a WARN result (Step 114). Subsequent to the WARN result, the method continues at Step 116, i.e., with knowledge that the pad electrodes are not ready for use and/or are defective and in need of replacement.

In another embodiment, the system 10 further comprises a battery connector sensor 37 in communication with the controller 24, the method further comprising the steps of: sensing (Step 118), via the battery connector sensor 37, an installation of a battery 36 into the system 10 by the battery connector sensor; initiating communications (Step 106), via the base unit 15 communication module 20, between the base unit 15 volatile data storage memory 40 and the electrode pad set 12 data storage memory 17 responsive to the battery installation sensing step; writing the data (Step 108) from the data storage memory 17 of the electrode pad set 12 to the base unit 15 volatile data storage memory 40; and subsequent to the writing step, performing (Step 110) an automatic electrode pad set self-test, via the self-testing circuit 25 and the lead wires 18, using the data. The method continues as discussed herein above with respect to Steps 112, 114 and 116.

In yet another embodiment, the system further comprises a display (e.g., user interface 22) in communication with the controller 24, the method 100 further comprising the step of: providing an indication (Step 120), via the display, that the writing step has occurred. According to one embodiment, the system 10 further comprises a second wireless communication module 21, the method further comprising the step of: transmitting (Step 122), via the second wireless communication module 21, a notification to a remote service provider 23 that the writing step has occurred.

With reference again to Figs. 1 and 2, according to a still further embodiment, a system 10 comprises: a controller 24; a self-testing circuit 25 under operational control of the controller 24 and disposed to automatically and periodically test a readiness of an electrode pad set 12; a volatile data storage memory 40 in communication with the self-testing circuit 25 and configured to store data pertaining to a relationship between a characteristic of an electrode gel and an environmental variable; and communication module 20 configured to receive the data pertaining to a relationship between a characteristic of the electrode gel and an environmental variable from a connected electrode pad set 12 and to store the data into the volatile data storage memory 40. Although only a few exemplary embodiments have been described in detail above, those skilled in the art will readily appreciate that many modifications are possible in the exemplary embodiments without materially departing from the novel teachings and advantages of the embodiments of the present disclosure. For example, the embodiments of the present disclosure can be advantageously used in patient monitor devices for monitoring a patient's ECG and/or other patient diagnostic characteristics, Automated External Defibrillators (AEDs), Defibrillators, and Transcutaneous Electrical Nerve Stimulation (TENS) devices. With respect to TENS devices, they are generally used for nerve related pain conditions (e.g., acute and chronic conditions) and operate by sending stimulating pulses across the surface of the skin and along the nerve strands. The stimulating pulses help prevent pain signals from reaching the brain. TENS devices also help stimulate a patient's body to produce higher levels of its own natural painkillers, called "endorphins". Accordingly, all such modifications are intended to be included within the scope of the embodiments of the present disclosure as defined in the following claims. In addition, any reference signs placed in parentheses in one or more claims shall not be construed as limiting the claims. The word "comprising" and "comprises," and the like, does not exclude the presence of elements or steps other than those listed in any claim or the specification as a whole. One or more of the embodiments may be implemented by means of hardware comprising several distinct elements, and/or by means of a suitably programmed computer. In a device claim enumerating several means, several of these means may be embodied by one and the same item of hardware. Measures recited in mutually different dependent claims may advantageously be used in combination.

## Claims

1. An electrode pad set (12) comprising:
a plurality of pad electrodes (14), each pad electrode including an electrode gel;
lead wires (18) for electrically coupling a respective pad electrode of the plurality of pad electrodes to a base unit (10) configured to use the electrode pad set;
an electrode pad set data storage memory (17) configured to store data relating to a characteristic of the electrode gel; and
a communication unit (16) configured to communicate the data relating to the characteristic of the electrode gel to the base unit (10) configured to use the electrode pad set (12).

2. The electrode pad set (12) of claim 1, wherein the data comprises at least one of a table of values of the characteristic of the electrode gel as relating to an environmental variable, and a functional relationship of the characteristic of the electrode gel as relating to the environmental variable.

3. The electrode pad set (12) of claim 1 or 2, wherein the characteristic of the electrode gel is electrical impedance and the environmental variable is one selected from temperature, a test signal frequency, and time.

4. The electrode pad set (12) of claim 3, wherein the data relating to the relationship further comprises a threshold criterion for an acceptable operating specification of the electrode gel.

5. A system (10) comprising:
an electrode pad set (12) according to any of the preceding claims; and
a base unit (15) comprising:
a communication module (20) for receiving from the communication unit (16), the data relating to the characteristic of the electrode gel,
a base unit data storage memory (40) configured to store the data received by the communication module (20), and
a socket (11) configured to receive lead wires (18) so as to provide electrical connection between the electrode pad set (12) and the base unit (10).

6. The system (10) according to claim 5, further comprising:
a connection sensor (27) configured to provide an electrode connection signal to a controller (24) responsive to a sensed connection between the socket (11) and the lead wires (18), wherein the controller (24) is configured to initiate a data transfer from the electrode pad set data storage memory (17) to the base unit data storage memory (40), responsive to the electrode connection signal.

7. The system (10) according to claim 6, wherein, subsequent to the data transfer, a self-testing circuit (25) automatically and periodically tests the readiness of the electrode pad set (12) using the transferred data.

8. The system (10) according to claim 6, further comprising a battery connector sensor (37) in communication with the controller (24), and configured to provide a battery connection signal responsive to a sensed installation of a battery (36) into the system, and further wherein the controller (24) initiates a data transfer from the electrode pad set data storage memory (17) to the base unit data storage memory (40), responsive to the battery connection signal.

9. The system (10) according to claim 7, further comprising a visual indicator and an audio output in communication with the controller (24), wherein at least one of the visual indicator and the audio output provide an alarm responsive to a sensed failed electrode pad set readiness test via the self-testing circuit.

10. The system (10) according to claim 6, wherein the transferred data comprises a table of values of the characteristic of the electrode gel as relating to the environmental variable, and further wherein, subsequent to the data transfer, the data in the base unit volatile data storage memory (40) is the same as the data in the electrode pad set data storage memory (17).

11. The system (10) according to claim 10, wherein the base unit data storage memory (40) is further configured to store at least one of electrode pad set test failure threshold criteria and upper and lower bounds for an electrode gel critical to quality characteristic.

12. A method for self-testing an electrode pad set of a system as claimed in any of the preceding claims 5-11, the method comprising the steps of:
sensing (104) a connection of the electrode pad set (12) to the base unit (15);
initiating communications (106) between the base unit data storage memory (40) and the electrode pad set data storage memory (17) of the responsive to the connection sensing step (104);
writing (108) data from the electrode pad set data storage memory (17) to the base unit data storage memory (40); and
subsequent to the writing step (108), performing (110) an automatic electrode pad set self-test, via self-testing circuit (25) and the lead wires (18), using the data.

13. The method of claim 12, wherein the system further comprises a battery connector sensor (37), the method comprising the steps of:
sensing (118), via the battery connector sensor (37), an installation of a battery (36) into the system;
initiating communications (106) between the base unit data storage memory (40) and the electrode pad set data storage memory (17) responsive to the battery installation sensing step (118);
writing (108) the data from the electrode pad set data storage memory (17) to the base unit data storage memory (40); and
subsequent to the writing step (108), performing (110) an automatic electrode pad set self-test, via the self-testing circuit (25) and the lead wires (118), using the data.

14. The method of claim 12, wherein the system further comprises a display in communication with the controller (24), the method further comprising the step of:
providing an indication, via the display, that the writing step (108) has occurred.

15. The method of claim 13 or 14, wherein the method further comprising the step of:
transmitting a notification to a remote service provider (23) that the writing step (108) has occurred.
